# EUROPEAN PATENT SPECIFICATION

(11) **EP 3 192 382 B1**
(45) Date of publication and mention of the grant of the patent: **05.04.2023**
(21) Application number: 17151777.4
(22) Date of filing: 17.01.2017
(51) Int. Cl.: A24F 40/485, A24F 40/10, A24F 40/40

(54) **ATOMIZER AND ELECTRONIC CIGARETTE**
VERDAMPFER UND ELEKTRONISCHE ZIGARETTE
DISPOSITIF D'ATOMISATION ET CIGARETTE ÉLECTRONIQUE

(30) Priority: 18.01.2016 CN 201620043887 U; 20.01.2016 CN 201620053006 U
(43) Date of publication of application: 19.07.2017
(73) Proprietor: Shenzhen First Union Technology Co., Ltd., Shenzhen, Guangdong 518100 (CN)
(72) Inventor: LI, Yonghai, Shenzhen, Guangdong 518100 (CN); XU, Zhongli, Shenzhen, Guangdong 518100 (CN); ZHAO, Xiaoqiang, Shenzhen, Guangdong 518100 (CN); YU, Guiyuan, Shenzhen, Guangdong 518100 (CN)
(74) Representative: ZHAOffice SPRL

(56) References cited:
- EP-A1- 2 614 731
- EP-A1- 2 856 892
- EP-A2- 3 143 884
- CN-A- 104 544 575
- CN-U- 203 388 273
- CN-U- 204 409 588
- CN-U- 204 444 245
- CN-U- 204 812 051
- CN-U- 204 949 516
- US-A1- 2014 217 056
- US-A1- 2015 250 340

## Description

### FIELD OF THE INVENTION

The present invention relates to the field of electronic cigarette, and more particularly, to an atomizer and an electronic cigarette with the atomizer.

### BACKGROUND OF THE INVENTION

As the electronic cigarette has advantages of safe-usage, convenience, health and environmental protection, more and more people pay attention to the electronic cigarette which is the replacement of an ordinary cigarette. The electronic cigarette includes an atomizer and a power supply component, an atomizing component and a liquid-storing component for storing the tobacco liquid are included in the atomizer, a vapor passage is arranged in the liquid-storing component, and the atomizing component further comprises a liquid-absorbing element and a heating element. The tobacco liquid in the liquid-absorbing element is atomized to form aerosol when the heating element heats, and then the aerosol flows through the vapor passage, thus an effect of a cigarette is achieved.

There are mainly two types of the atomizer in the prior art, which are a disposable atomizer and a refillable atomizer with tobacco liquid. The usage cost may be reduced, as the refillable atomizer can be recycled. Therefore, an atomizer, into which the tobacco liquid can be injected conveniently, is needed.

An EP patent application EP2856892A1 discloses a suction rod comprising a suction nozzle cover and a connection assembly respectively inserted into both ends of a suction cylinder, a guide tube disposed within the suction cylinder, a liquid-guiding assembly and an atomization device, wherein both ends of a cigarette liquid bin are enclosed respectively by the suction nozzle cover and the liquid-guiding assembly which are provided with central through-holes, and the guide tube is provided within the cigarette liquid bin such that both ends of the guide tube are respectively inserted into the central though-holes of the suction nozzle cover and of the liquid-guiding assembly, with the interiors of the suction nozzle cover, the liquid-guiding assembly and the guide tube being in communication with each other and forming a smoke passage; the atomization device is provided between the liquid-guiding assembly and the connection assembly; the liquid-guiding assembly comprises a liquid-separation base and a liquid-storing body, with the liquid-separation base comprising a base body and a ventilation tube; the central through-hole of the liquid-guiding assembly is defined within the ventilation tube; one end of the ventilation tube is matched to the guide tube, and the other end thereof is sheathed inside the liquid-storing body and leads to the atomization device; and a liquid-guiding hole is provided on the liquid-separation base.

An EP patent application EP2614731A1 discloses an atomization shell which is a hollow pipe with its first end connected to the mouth-piece and its second end sealed by a sealing sleeve and the thread bushing. An aerosol passage is provided inside the atomization shell from its first end. The aerosol passage has one end in communication with an orifice provided on the mouth-piece and another end communicating with the sealing sleeve. The sealing sleeve is muff-coupled to the thread bushing. The atomization shell further has a first intermediary piece therein which extends into the sealing sleeve for introducing tobacco liquid stored in the atomization shell into the sealing sleeve. And an electric heating element is provided between the sealing sleeve and the thread bushing. The electric heating element, upon powered, heats up the tobacco liquid immerged in the first intermediary piece to make it atomized quickly and the atomized tobacco liquid flows through the aerosol passage and is discharged through the mouth-piece to human mouth. This atomizer adopts sealing sleeve and the atomization shell to form a storage room which improves the tobacco liquid reservation capability of the electronic cigarette and simplify the manufacturing of the cigarette.

A CN patent application CN204409588U discloses an electronic cigarette atomizing device which comprises a liquid storage cup for storing tobacco liquid, an outer tube arranged on the liquid storage cup and a heating atomizing component arranged at the bottom of the liquid storage cup. The bottom of the heating atomizing component is sealed through a liquid blocking plug, a top cover and a sealing plug both used for sealing the top of the liquid storage cup are detachably arranged on the top of the liquid storage cup, the liquid storage cup is provided with a injection hole and an exhaust hole, and a injection hole plug and an exhaust hole plug are correspondingly arranged on the inner side of the top cover.

A CN patent application CN203388273U discloses an electronic cigarette comprising an atomizing assembly provided with an outer sleeve, wherein an oil storage space is arranged in the atomizing assembly, a suction nozzle cover movably connected with the outer sleeve is inserted into one end of the outer sleeve, an oiling opening used for injecting cigarette oil is formed in the side face of the suction nozzle cover, and the oiling opening is located in the inner side of the outer sleeve and is communicated with the oil storage space.

CN utility model application CN204949516 discloses an atomizer and electronic cigarette, wherein, the atomizer is connected with power supply unit in the electron cigarette. This atomizer includes: base, base include the lower cover, install in the atomizing core of lower cover and cup joint in the baffle of atomizing core, the sleeve, the sleeve is connected with the lower cover, and atomizing core and baffle holding are in the sleeve, upper cover, upper cover and sleeve enclose with the base and close the formation cavity, and the baffle separates the cavity for spray chamber and oil storage chamber, and the upper cover is equipped with the oiling mouth that communicates with the oil storage chamber, the suction nozzle, suction nozzle and spray chamber intercommunication. The entire cover including the mouthpiece are rotatable relative to the atomizer core for refilling the reservoir.

CN utility model application CN204812051 discloses an electronic cigarette atomizer comprising an outer tube, an upper oil inlet cover, an upper cover fixing member, an atomizing sleeve, an atomizing core, a flue assembly and a base fixing assembly; wherein the upper cover fixing member and the base fixing components are respectively installed at both ends of the outer tube, the atomizing sleeve is sleeved in the outer tube and the lower end of the atomizing sleeve is clamped between the outer tube and the base fixing component, an oil storage cavity is formed between the outer wall of the flue assembly and the inner wall of the outer tube; the bottom end of the flue assembly is fixedly connected with the atomization sleeve, and the top end of the flue assembly is fixedly connected with the upper cover fixing member; the atomization core is sleeved in the atomization sleeve and fixedly connected with the base fixing component; the upper cover fixing part is provided with a sliding groove and the sliding groove is provided with an oil injection hole communicating with the oil storage cavity; the oil inlet upper cover is provided with The sliding block is matched with the sliding groove; the sliding block is slidably arranged in the sliding groove and shielding the oil injection hole, and pushing the sliding block to expose the oil injection hole for filling smoke oil.

### SUMMARY OF THE INVENTION

An atomizer and an electronic cigarette are disclosed in the invention to inject tobacco liquid into the atomizer more conveniently.

In accordance with one aspect of the invention, an atomizer with the features of claim 1 is disclosed, comprises: a housing with a first end and a second end; a mouthpiece, connected to the first end of the housing, wherein the second end (102) is configured to be connected to a power supply (200) of an electronic cigarette; a reservoir, formed in the housing and configured to contain tobacco liquid; and an atomizing element, configured to atomize the tobacco liquid to form aerosol; wherein an injecting hole and a vent hole are formed on the first end of the housing, the injecting hole and the vent hole are in communication with the reservoir, the atomizer further comprises a cover movably connected to the first end of the housing, and the cover is movable with respect to the first end to expose or cover the injecting hole and the vent hole; wherein the first end of the housing is stepped, and the cover has a shape corresponding to a stepped location of the first end of the housing such that the cover is containable in the stepped location of the first end of the housing and the cover is arranged to be movable independently from the mouthpiece to expose or cover the injecting hole and the vent hole by sliding or rotating.

Wherein, in a preferred embodiment the cover is rotatable in relative to the first end of the housing, to expose or cover the injecting hole and the vent hole, and the atomizer further comprises a rotation shaft formed on the first end of the housing, and the cover is pivoted along the rotation shaft such that the cover is rotatable in relative to the first end of the housing.

Wherein, in a preferred embodiment the cover is slidable with respect to the first end of the housing, to expose or cover the injecting hole and the vent hole, and two sliding slots are formed on any one of the first end of the housing and the cover, two protruded guiding elements are formed on another of the first end of the housing and the cover, and the two protruded guiding elements are inserted into the two sliding slots respectively and slidable along the two sliding slots such that the cover is slidable along the radial direction of the first end of the housing.

Wherein, in a preferred embodiment the two protruded guiding elements are fixedly formed on the first end of the housing or the cover via being inserted into two first fixed holes formed on the first end of the housing or the cover, respectively.

Wherein, in a preferred embodiment the cover is slidable with respect to the first end of the housing to expose or cover the injecting hole and the vent hole and further comprising: a positioning structure for hinting the first end of the housing is tightly closed by the cover; wherein the positioning structure comprises two deformation elements and two matching elements formed on the cover and the first end of the housing respectively, when a matching relation is formed between the two deformation elements and the two matching element, the stepped location of the first end of the housing is tightly closed by the cover; and when the matching relation is released, the cover exposes at least a part of the stepped location of the first end of the housing.

Wherein, in a preferred embodiment the two deformation elements are fixedly formed on the cover or the first end of the housing via being inserted into two second fixed holes formed on the cover or the first end of the housing, respectively.

Wherein, in a preferred embodiment the two deformation elements are elastic thimbles respectively, and the two matching elements are performed by the two sliding slots formed on the first end of the housing or the cover.

Wherein, in a preferred embodiment each of the two deformation elements comprises a spring and a top element arranged on the spring.

Wherein, in a preferred embodiment a seal ring is further arranged on the first end or the cover and around the injecting hole and the vent hole, for forming a sealing connection between the cover and the first end of the housing when the cover covers the injecting hole and the vent hole, to avoid the tobacco liquid leaking.

Wherein, in a preferred embodiment the injecting hole has an aperture larger than that of the vent hole.

Wherein, in a preferred embodiment a rugged portion is formed on the cover for enhancing a users' touch.

In accordance with another aspect of the invention, an electronic cigarette is disclosed, comprising: an atomizer as described in any of the claims 1-13; and a power supply, configured to supply electric power to the atomizer.

Beneficial effects of the invention may be as follows: the injecting hole and the vent hole are formed on the atomizer, which may be exposed or covered by the cover. Users may only move the cover to expose the injecting hole and the vent hole, when injecting tobacco liquid into the atomizer. Therefore, it is more convenient to operate.

### BRIEF DESCRIPTION OF THE DRAWINGS

In order to better illustrate technical implementations of the invention, hereinafter the accompanying drawings used in embodiments will be described simply. Obviously, the drawings as described are only some embodiments. All other drawings shall be obtained by those of skill in the art based on those drawings without making inventive efforts. Herein:
FIG. 1 is a structural view illustrating an atomizer in accordance with a first embodiment of the invention;
FIG. 2 is a schematic cutaway view of the atomizer in accordance with the first embodiment in FIG. 1;
FIG. 3 is a top view of the atomizer in accordance with the first embodiment in FIG. 1;
FIG. 4 is a structural view illustrating an electronic cigarette in accordance with a first embodiment of the invention;
FIG. 5 is a structural view illustrating an atomizer in accordance with a second embodiment of the invention;
FIG. 6 is a structural view of the atomizer when a cover is opened in accordance with the second embodiment of the invention;
FIG. 7 is a schematic cutaway view of the atomizer in accordance with the second embodiment in FIG. 5;
FIG. 8 is a partial structural view of the atomizer in accordance with the second embodiment in FIG. 5;
FIG. 9 is a disassembled view of a cover in accordance with the second embodiment in FIG. 5;
FIG. 10 is a structural view illustrating an electronic cigarette in accordance with a second embodiment of the invention.

### DETAILED DESCRIPTION OF THE INVENTION

Now technical implementations of the invention will be described in definite and comprehensive details with reference to the accompanying drawings. Obviously, the embodiments as described are only some but not all the embodiments of the invention. All other embodiments obtained by those of skill in the art based on the embodiments of the invention without making inventive efforts shall all fall in the protection of the invention as defined by the appended claims.

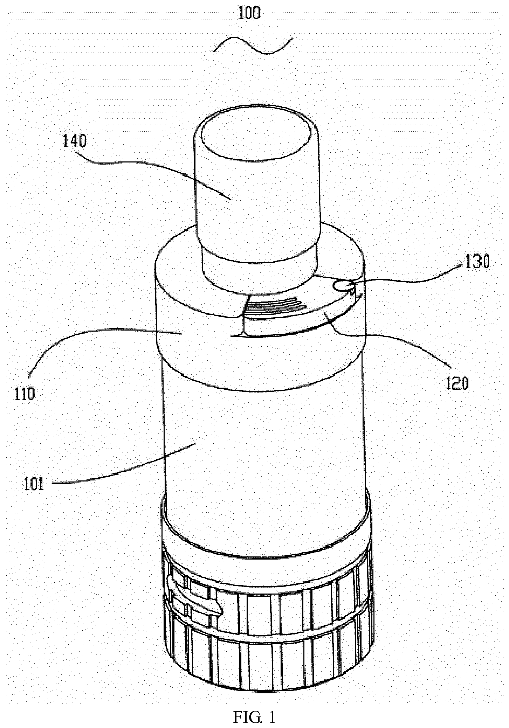

An atomizer in accordance with a first embodiment will be described with reference to FIGs.1 to 3 below.

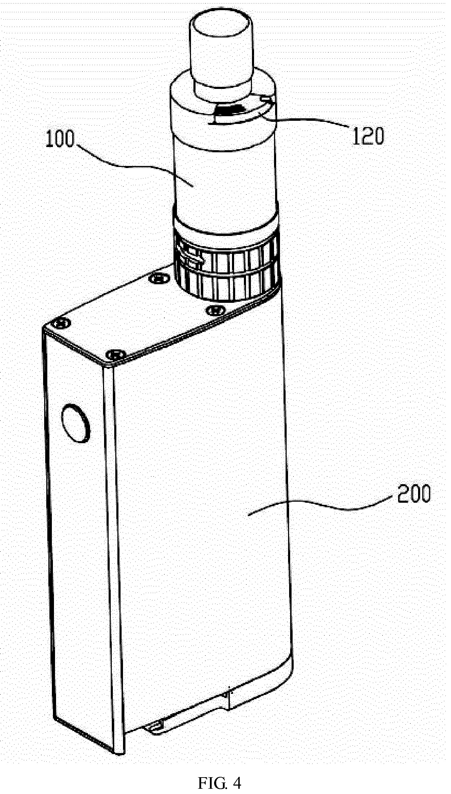

Referring to FIGS. 1-3, the atomizer 100, which is combined with a power supply 200 (as shown in FIG. 4) to form an electronic cigarette, includes a housing 101 with a first end 110 and a second end 102, and a mouthpiece 140 connected to the first end 110 of the housing 101. A reservoir 150 configured to contain tobacco liquid and an atomizing element 160 configured to atomize the tobacco liquid to form aerosol are formed inner side of the housing 101. An injecting hole 111 and a vent hole 112 are formed on the first end 110, and the injecting hole 111 and the vent hole 112 are both communicated with the reservoir 150. A cover 120 is formed on the first end 110. The cover 120 is movable with respect to the first end 110 to expose and cover the injecting hole 110 and the vent hole 112. Further, the first end 110 is stepped, and the cover 120 has a shape corresponding to a stepped location of the first end 110 of the housing 101 such that the cover 120 is capable of being contained in the stepped location of the first end 110.

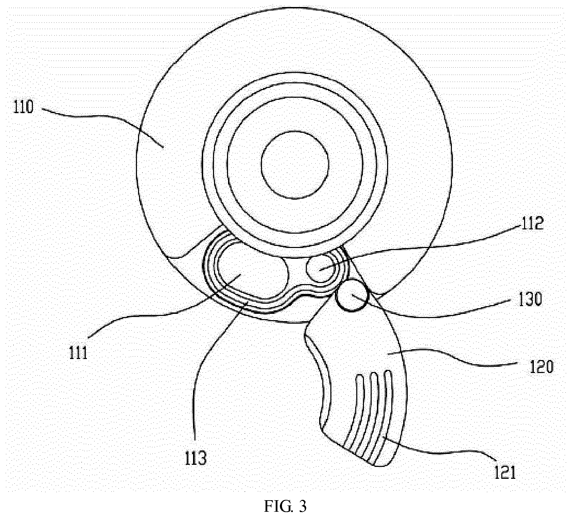

In the first embodiment, the cover 120 is rotatable in relative to the first end 110 to expose or cover the injecting hole 111 and the vent hole 112. Further, as shown FIG. 1 and FIG. 3, a rotation shaft 130 is formed on the first end 110, and the cover 120 is pivoted along the rotation shaft 130, thus the cover 120 may be rotatable in relative to the first end 110 to expose and cover the injecting hole 110 and the vent hole 112. Preferably, the rotation shaft 130 is formed near one edge of the stepped location of the first end 110 of the housing 101 where the vent hole 112 is formed, in this way, it is conveniently to inject tobacco liquid.

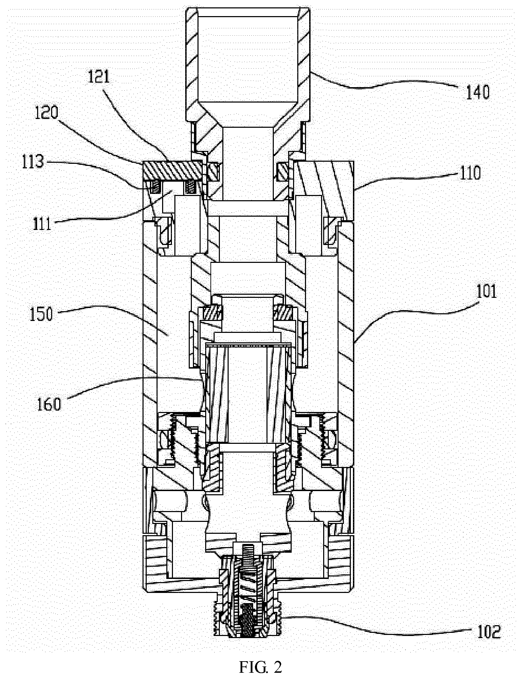

In the first embodiment, as shown in FIG. 2, the mouthpiece 140 is detachably connected to the first end 110. It is understood that the connection between the mouthpiece 140 and the first end 110 may also be in another manner, the mouthpiece 140 is fixed to the first end 110, for example in another embodiment.

As shown in FIGS. 2 and 3, a seal ring 113 is further arranged on the first end 110 and around the injecting hole 111 and the vent hole 112, which is configured to form a sealing connection between the cover 120 and the first end 110 when the cover 120 covers the injecting hole 111 and the vent hole 112 to avoid the tobacco liquid leaking. The seal ring 113 is made of rubber or silica gel material, in this way, a good sealing effect for the sealing connection between the cover 120 and the first end 110 may be achieved. Similarly, in another embodiment, the seal ring 113 may also be arranged on the cover 120. When the seal ring 113 is arranged on the cover 120, and around the injecting hole 111 and the vent hole 112. The seal ring 113 is configured to form a sealing connection between the cover 120 and the first end 110 when the cover 120 covers the injecting hole 111 and the vent hole 112 to avoid the tobacco liquid leaking.

Further, as shown in FIG. 3 again, the injecting hole 111 has an aperture larger than that of the vent hole 112.

As shown in FIGS. 2 and 3 again, a rugged portion 121 is further formed on the cover 120 for enhancing a users' touch. When opening the cover 120, the rugged portion 121 is helpful to enhance the touch between fingers and the cover 120.

As it can be seen, in the first embodiment, the injecting hole 111 and the vent hole 112 are formed on the atomizer 100, which are to be exposed or covered in an rotatable manner, therefore, users may only need to expose the injecting hole 111 and the vent hole 112 in the rotatable manner, when injecting tobacco liquid into the atomizer 100, thus it is more convenient to operate.

As shown in FIG. 4 which illustrates an electronic cigarette in accordance with a first embodiment of the invention, the electronic cigarette comprises an atomizer 100 and a power supply 200. The atomizer 100 is connected to the power supply 200 through a detachable structure, such as a detachable threaded connection structure. The power supply 200 is configured to provide the atomizer 100 for electric power, and the atomizer 100 is one as described in the above first embodiment with reference to the accompanying FIGS. 1-3.

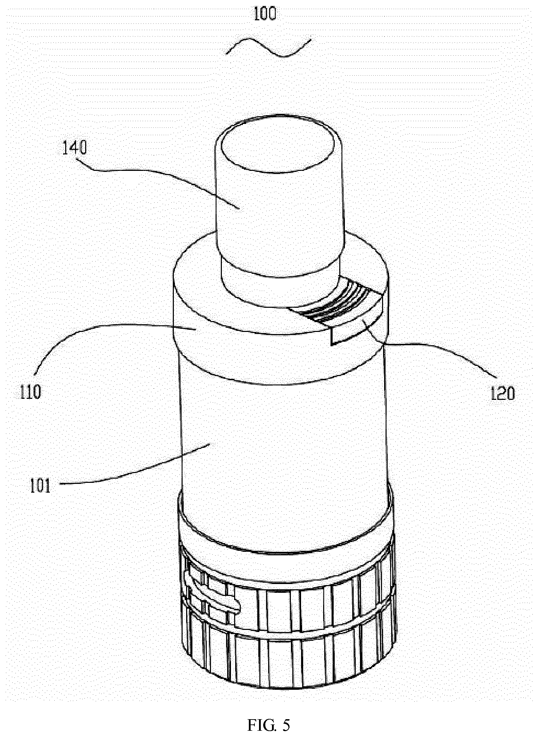

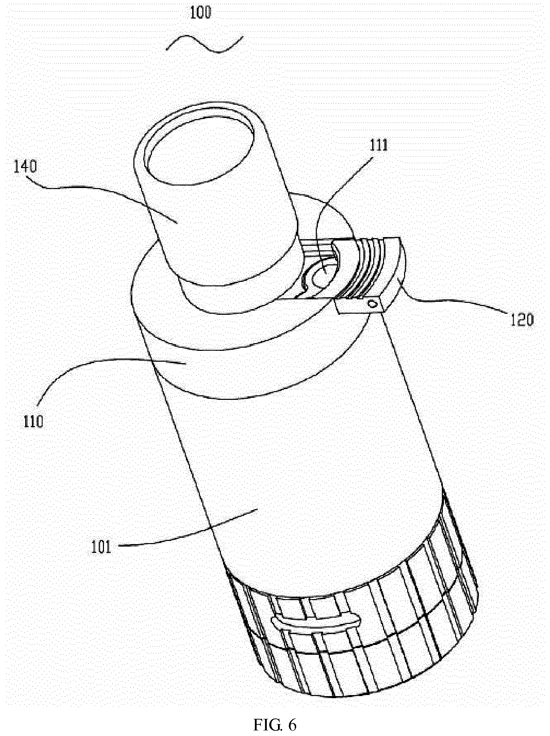

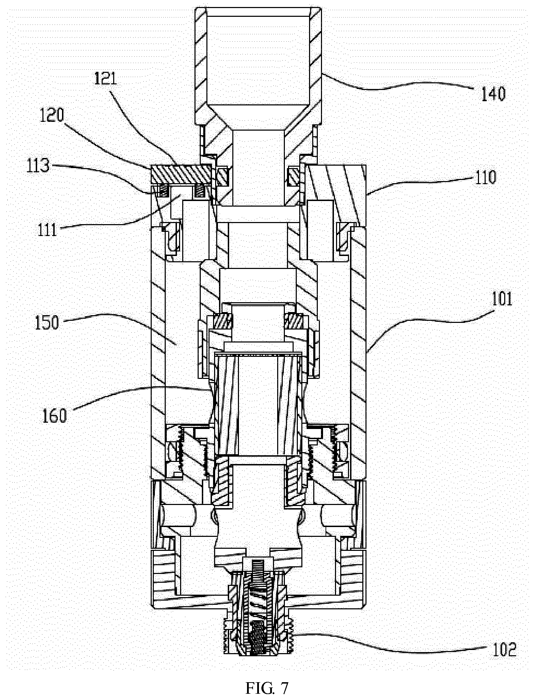

An atomizer in accordance with a second embodiment will be described with reference to FIGs.5 to 8 below.

The difference between atomizers in the first embodiment and the second embodiment is that the cover 120 is slidable with respect to the first end 110 to expose or cover the injecting hole 111 and the vent hole 112. The remaining technical characteristics of the atomizer 100 in the second embodiment are same to those in the first embodiment, which are not to be described in details again here. Now the difference will be described in detail.

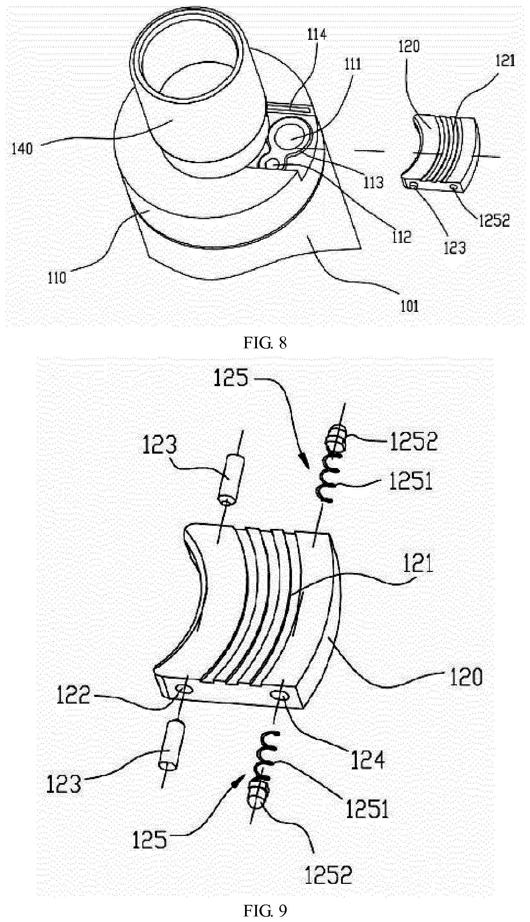

As shown in FIG. 8, in the second embodiment, the first end 110 is circular-shaped, and the cover 120 is slidable along a radial direction of the first end 110. Two sliding slots 114 is formed on the first end 110, and two guiding elements 123 are formed on both sides of the cover 120, respectively. The two guiding elements 123 are inserted into the two sliding slots 114 respectively and slidable along the two sliding slots 114 such that the cover 120 is slidable along the radial direction of the first end 110. The guiding element 123 is a cylindrical object. One end of one guiding element 123 is protruded at one side of the cover 120. One end of the other guiding element 123 is protruded at the other side of the cover 120. The guiding elements 123 are adapted to the sliding slots 114, and the guiding element 123 is always in the slide slot 114.

Further, two first fixed holes 122 are formed on the cover 120. The two guiding elements 123 are fixedly formed on the cover 120 via being inserted into the two first fixed holes 122, one ends of which are protruded from both sides of the cover 120, respectively. In this way, the guiding elements 123 are formed on the cover 120. What may be understood for the skilled in the art, the guiding element 123 may also be formed on the first end 110, and the slide slot 114 may also be formed on the cover 120. In this way, the two first fixed holes 122 are formed on the first end 110, and the two guiding elements 123 are fixedly formed on the first end 110 via being inserted into the two first fixed holes 122.

As shown in FIG. 9, the atomizer 100 further includes a positioning device for hinting the first end 110 is tightly closed by the cover 120. The positioning device includes two deformation elements 125 and two matching elements 114. The two deformation elements 125 are formed on the cover 120, and the two matching elements 114 are formed on the first end 110. When a matching relation is formed between the two deformation elements 125 and the two matching element 114, the stepped location of the first end 110 is tightly closed by the cover 120; and when the matching relation is released, the cover 120 exposes at least a part of the stepped location of the first end 110.

Further, two second fixed holes 124 are formed on the cover 120. The two deformation elements 125 are fixedly formed on the cover 120 via being inserted into two second fixed holes 124 formed on the cover 120. In this way, the deformation elements 125 are formed on the cover 120. What may be understood for the skilled in the art, the deformation elements 125 may also be formed on the first end 110. In this way, the two second fixed holes 124 are formed on the first end 110, and the two deformation elements 125 are fixedly formed on the first end 110 via being inserted into the two second fixed holes 124.

In the second embodiment, the two deformation elements 125 are elastic thimbles respectively, and the matching elements 114 are performed by the two sliding slots 114 formed on the first end 110. The elastic thimbles are matched with the two sliding slots 114, respectively. It should be state that the matching element 114 and the slide slot 114 are integrated in this embodiment, thus the deformation element 125 is matched with the slide slot 114. What may be understood the matching element 114 and the slide slot 114 may also be formed separately.

The elastic thimble includes a top element 1252 and a spring 1251. The top element 1252 is arranged on the spring 1251. The top element 1252 is abutted against the slot when the first end 110 is tightly closed by cover 120.

As it can be seen, in the second embodiment, the injecting hole 111 and the vent hole 112 are formed on the atomizer 100, to be exposed or covered in a slidable manner, therefore, users may only need to expose the injecting hole 111 and the vent hole 112 through sliding the cover 120, when injecting tobacco liquid into the atomizer 100, thus it is more convenient to operate.

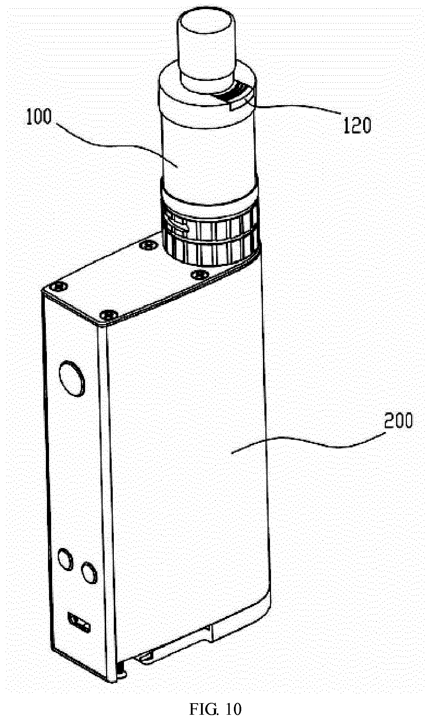

As shown in FIG. 10 which illustrates an electronic cigarette in accordance with a second embodiment of the invention, the electronic cigarette includes an atomizer 100 and a power supply 200. The atomizer 100 is connected to the power supply 200 through a detachable structure, such as a detachable threaded connection structure. The power supply 200 is configured to provide the atomizer 100 for electric power, and the atomizer 100 is one as described in the above second embodiment with reference to the accompanying FIGS. 5-8.

In conclusion, the injecting hole and the vent hole are formed on the atomizer, which may be exposed or covered by the cover. Users may only move the cover to expose the injecting hole and the vent hole, when injecting tobacco liquid into the atomizer 100. Therefore, it is more convenient to operate.

## Claims

1. An atomizer (100), comprising:
a housing (101) with a first end (110) and a second end (102);
a mouthpiece (140), connected to the first end (110) of the housing (101), wherein the second end (102) is configured to be connected to a power supply (200) of an electronic cigarette;
a reservoir (150), formed in the housing (101) and configured to contain tobacco liquid; and
an atomizing element (160), configured to atomize the tobacco liquid to form aerosol;
wherein an injecting hole (111) and a vent hole (112) are formed on the first end (110) of the housing (101), the injecting hole (111) and the vent hole (112) are in communication with the reservoir (150), the atomizer (100) further comprises a cover (120) movably connected to the first end (110) of the housing (101), and the cover (120) is movable with respect to the first end (110) to expose or cover the injecting hole (111) and the vent hole (112);
wherein the first end (110) of the housing (101) is stepped, and the cover (120) has a shape corresponding to a stepped location of the first end (110) of the housing (101) such that the cover (120) is capable of being contained in the stepped location of the first end (110) of the housing (101) the atomizer being **characterised in that** the cover (120) is arranged to be movable independently from the mouthpiece (140) to expose or cover the injecting hole (111) and the vent hole (112) by sliding or rotating.

2. The atomizer (100) according to claim 1, wherein the cover (120) is rotatable in relative to the first end (110) of the housing (101), to expose or cover the injecting hole (111) and the vent hole (112), and the atomizer (100) further comprises a rotation shaft (130) formed on the first end (110) of the housing (101), and the cover (120) is pivoted along the rotation shaft (130) such that the cover (120) is rotatable in relative to the first end (110) of the housing (101).

3. The atomizer (100) according to claim 1, wherein the cover (120) is slidable with respect to the first end (110) of the housing (101), to expose or cover the injecting hole (111) and the vent hole (112), and wherein two sliding slots (114) are formed on any one of the first end (110) of the housing (101) and the cover (120), two protruded guiding elements (123) are formed on another of the first end (110) of the housing (101) and the cover (120), and the two protruded guiding elements (123) are inserted into the two sliding slots (114) respectively and slidable along the two sliding slots (114) such that the cover (120) is slidable along the radial direction of the first end (110) of the housing (101).

4. The atomizer (100) according to claim 3, wherein the two protruded guiding elements (123) are fixedly formed on the first end (110) of the housing (101) or the cover (120) via being inserted into two first fixed holes (122) formed on the first end (110) of the housing (101) or the cover (120), respectively.

5. The atomizer (100) according to claim 1, wherein the cover (120) is slidable with respect to the first end (110) of the housing (101)to expose or cover the injecting hole (111) and the vent hole (112) and further comprising:
a positioning structure for hinting the first end (110) of the housing (101) is tightly closed by the cover (120);
wherein the positioning structure comprises two deformation elements (125) and two matching elements (114) formed on the cover (120) and the first end (110) of the housing (101) respectively, when a matching relation is formed between the two deformation elements (125) and the two matching elements (114), the stepped location of the first end (110) of the housing (101) is tightly closed by the cover (120); and when the matching relation is released, the cover (120) exposes at least a part of the stepped location of the first end (110) of the housing (101).

6. The atomizer (100) according to claim 5, wherein the two deformation elements (125) are fixedly formed on the cover (120) or the first end (110) of the housing (101) via being inserted into two second fixed holes (124) formed on the cover (120) or the first end (110) of the housing (101), respectively.

7. The atomizer (100) according to claim 6, wherein the two deformation elements (125) are elastic thimbles respectively, and the two matching elements (114) are performed by the two sliding slots (114) formed on the first end (110) of the housing (101) or the cover (120).

8. The atomizer (100) according to claim 7, wherein each of the two deformation elements (125) comprises a spring (1251) and a top element (1252) arranged on the spring (1251).

9. The atomizer (100) according to claim 1, wherein a seal ring (113) is further arranged on the first end (110) or the cover (120) and around the injecting hole (111) and the vent hole (112), for forming a sealing connection between the cover (120) and the first end (110) of the housing (101) when the cover (120) covers the injecting hole (111) and the vent hole (112), to avoid the tobacco liquid leaking.

10. The atomizer (100) according to claim 1, wherein the injecting hole (111) has an aperture larger than that of the vent hole (112).

11. The atomizer (100) according to claim 1, wherein a rugged portion (121) is formed on the cover (120) for enhancing a users' touch.

12. An electronic cigarette, comprising:
an atomizer (100) of any one of claims 1-11; and
a power supply (200), configured to supply electric power to the atomizer (100).

## Patentansprüche

1. Verdampfer (100), umfassend:
ein Gehäuse (101) mit einem ersten Ende (110) und einem zweiten Ende (101);
ein Mundstück (140) mit dem ersten Ende (110) des Gehäuses (101) verbunden, wobei das zweite Ende (102) zur Verbindung mit einer Stromversorgung (200) einer elektronischen Zigarette konfiguriert ist;
einen Behälter (150), der im Gehäuse gebildet ist und zur Beinhaltung von Tabakflüssigkeit konfiguriert ist;
ein Verdampfelement (160), das zur Verdampfung der Tabakflüssigkeit konfiguriert ist, um ein Aerosol zu bilden;
wobei ein Einspritzloch (111) und ein Entlüftungsloch (112) auf dem ersten Ende des Gehäuses (101) gebildet sind, das Einspritzloch (111) und das Entlüftungsloch (112) in Verbindung mit dem Behälter (150) stehen, der Verdampfer (100) weiter einen Deckel (120) umfasst, der beweglich mit dem ersten Ende (110) des Gehäuses (101) verbunden ist, und der Deckel (120) beweglich gegenüber dem ersten Ende (110) ist, um das Einspritzloch (111) und das Entlüftungsloch (112) auszusetzen oder zu bedecken;
wobei das erste Ende (110) des Gehäuses (101) gestuft ist, und der Deckel (120) eine Gestalt aufweist, die einem gestuften Ort des ersten Endes (110) des Gehäuses (101) derart entspricht, dass der Deckel (120) im gestuften Ort des ersten Endes (110) des Gehäuses (101) enthalten sein kann,
wobei der Verdampfer (100) **dadurch gekennzeichnet ist, dass** der Deckel (120) so angeordnet ist, dass er unabhängig von dem Mundstück beweglich ist, um das Einspritzloch (111) und das Entlüftungsloch (112) durch Verschiebung oder Drehung auszusetzen oder zu bedecken.

2. Verdampfer (100) nach Anspruch 1, wobei der Deckel (120) drehbar gegenüber dem ersten Ende (110) des Gehäuses (101) ist, um das Einspritzloch (111) und das Entlüftungsloch (112) auszusetzen oder zu bedecken, und der Verdampfer (100) weiter eine auf dem ersten Ende (110) des Gehäuses (101) gebildeten Drehwelle umfasst, und der Deckel (120) die Drehwelle (130) entlang so dreht, dass der Deckel (120) gegenüber dem ersten Ende (110) des Gehäuses (101) drehbar ist.

3. Verdampfer (100) nach Anspruch 1, wobei der Deckel (120) gegenüber dem ersten Ende (110) des Gehäuses (101) verschiebbar ist, um das Einspritzloch (111) und das Entlüftungsloch (112) auszusetzen oder zu bedecken, und wobei zwei Schiebenuten (114) auf einem Element aus dem ersten Ende (110) des Gehäuses (101) und dem Deckel (120) gebildet sind, zwei herausgeragte Führungselemente (123) auf einem anderen Element aus dem ersten Ende (110) des Gehäuses (101) und dem Deckel (120) gebildet sind, und die zwei herausgeragten Führungselemente (123) jeweils in den zwei Schiebenuten (114) eingesetzt sind und die radiale Richtung des ersten Ende (110) des Gehäuses (101) entlang derart verschiebbar sind, dass der Deckel (120) die radiale Richtung des ersten Ende (110) des Gehäuses (101) entlang verschiebbar ist.

4. Verdampfer (100) nach Anspruch 3, wobei die zwei herausgeragten Führungselemente (123) fest auf dem ersten Ende (110) des Gehäuses (101) oder dem Deckel (120) gebildet sind, indem sie jeweils in zwei ersten festen Löchern (122), die auf dem ersten Ende (110) des Gehäuses (101) oder auf dem Deckel (120) gebildet sind, eingesetzt sind.

5. Verdampfer (100) nach Anspruch 1, wobei der Deckel (120) gegenüber dem ersten Ende (110) des Gehäuses (101) verschiebbar ist, um das Einspritzloch (111) und das Entlüftungsloch (112) auszusetzen oder zu bedecken, und weiter umfasst:
eine Positionierungsstruktur zur Andeutung des ersten Ende (110) des Gehäuses (101) ist durch den Deckel (120) dicht geschlossen;
wobei die Positionierungsstruktur zwei Verformungselemente (125) und zwei Anpassungselemente (114) umfasst, die jeweils auf dem Deckel (120) und auf dem ersten Ende (110) des Gehäuses (101) gebildet sind, wenn eine Anpassungsverbindung zwischen den zwei Verformungselementen (125) und den zwei Anpassungselementen (114) gebildet ist, der gestufte Ort des ersten Ende (110) des Gehäuses (101) durch den Deckel (120) dicht geschlossen ist, und wenn die Anpassungsverbindung gelöst ist, der Deckel (120) zu mindestens ein Teil des gestuften Ortes des ersten Endes (110) des Gehäuses (101) aussetzt.

6. Verdampfer (100) nach Anspruch 5, wobei die zwei Verformungselemente (125) fest auf dem Deckel (120) oder auf dem ersten Ende (110) des Gehäuses (101) gebildet sind, indem sie jeweils in zwei zweiten festen Löchern (124), die auf dem Deckel (120) oder auf dem ersten Ende (110) des Gehäuses (101) gebildet sind, eingesetzt sind.

7. Verdampfer (100) nach Anspruch 6, wobei die zwei Verformungselemente (125) jeweils kleine elastische Rohre sind, und die zwei Anpassungselemente (114) durch zwei Schiebenuten (114) ausgeführt sind, die auf dem ersten Ende (110) des Gehäuses (101) oder auf dem Deckel (120) gebildet sind.

8. Verdampfer (100) nach Anspruch 7, wobei jedes der zwei Verformungselemente (125) eine Feder (1251) und ein Oberelement (1252) umfasst, das auf der Feder (1251) angeordnet ist.

9. Verdampfer (100) nach Anspruch 1, wobei ein Dichtring (113) weiter auf dem ersten Ende (110) oder auf dem Deckel (120) um das Einspritzloch (111) und das Entlüftungsloch (112) angeordnet ist, um eine Dichtverbindung zwischen dem Deckel (120) und dem ersten Ende (110) des Gehäuses (101) zu bilden, wenn der Deckel (120) das Einspritzloch (111) und das Entlüftungsloch (112) zur Vermeidung eines Tabakflüssigkeitslecks bedeckt.

10. Verdampfer (100) nach Anspruch 1, wobei das Einspritzloch (111) eine Öffnung größer als diese des Entlüftungslochs (112) aufweist.

11. Verdampfer (100) nach Anspruch 1, wobei ein raues Teil (121) auf dem Deckel (120) zur Erhöhung des Benutzergefühls gebildet ist.

12. Elektronische Zigarette, umfassend:
einen Verdampfer (100) nach einem der Ansprüche 1 - 11; und eine Stromversorgung (200), die zur Versorgung elektrischer Energie zum Verdampfer (100) konfiguriert ist.

## Revendications

1. Dispositif d'atomisation (100), comprenant :
un boîtier (101) présentant une première extrémité (110) et une deuxième extrémité (102) ;
un embout (140), raccordé à la première extrémité du boîtier (101), où la deuxième extrémité (102) est configurée pour être raccordée à une alimentation électrique (200) d'une cigarette électronique ;
un réservoir (150), formé dans le boîtier (101) et configuré pour contenir du tabac liquide ; et
un élément atomiseur (160), configuré pour atomiser le tabac liquide afin de former un aérosol ;
dispositif dans lequel un orifice d'injection (111) et un orifice d'évent (112) sont formés sur la première extrémité (110) du boîtier (101), l'orifice d'injection (111) et l'orifice d'évent (112) sont en communication avec le réservoir (150), le dispositif d'atomisation (100) comprend en outre un couvercle (120) raccordé de façon mobile à la première extrémité (110) du boîtier (101), et le couvercle (120) est mobile par rapport à la première extrémité afin d'exposer ou de couvrir l'orifice d'injection (111) et l'orifice d'évent (112) ;
où la première extrémité (110) du boîtier (101) est en gradin, et le couvercle (120) présente une forme correspondant à un endroit en gradin de la première extrémité (110) du boîtier (101) de sorte que le couvercle (120) soit apte à être contenu dans l'endroit en gradin de la première extrémité (110) du boîtier (101),
le dispositif d'atomisation (100) étant **caractérisé en ce que** le couvercle (120) est disposé de façon à être mobile indépendamment de l'embout (140) afin d'exposer ou de couvrir l'orifice d'injection (111) et l'orifice d'évent (112) par coulissement ou rotation.

2. Dispositif d'atomisation (100) selon la revendication 1, dans lequel le couvercle (120) est rotatif par rapport à la première extrémité (110) du boîtier (101) afin d'exposer ou de couvrir l'orifice d'injection (111) et l'orifice d'évent (112), et le dispositif d'atomisation (100) comprend en outre un arbre rotatif (130) formé sur la première extrémité (110) du boîtier (101), et le couvercle (120) pivote le long de l'arbre rotatif (130) par rapport à la première extrémité (110) du boîtier (101).

3. Dispositif d'atomisation (100) selon la revendication 1, dans lequel le couvercle (120) peut coulisser par rapport à la première extrémité du boîtier (101) afin d'exposer ou de couvrir l'orifice d'injection (111) et l'orifice d'évent (112), et dans lequel deux rainures de coulissement (114) sont formées sur n'importe quel élément parmi la première extrémité du boîtier (101) et le couvercle (120), et deux éléments saillants de guidage (123) sont formés sur un autre élément parmi la première extrémité du boîtier (101) et le couvercle (120), et les deux éléments saillants de guidage (123) sont insérés respectivement dans les deux rainures de coulissement (114) et sont coulissables le long des deux rainures de coulissement (114) de sorte que le couvercle (120) puisse coulisser le long de la direction radiale de la première extrémité (110) du boîtier (101).

4. Dispositif d'atomisation (100) selon la revendication 3, dans lequel les deux éléments saillants de guidage (123) sont formés de façon fixe sur la première extrémité (110) du boîtier (101) ou sur le couvercle (120) en étant insérés dans deux premiers orifices fixes (122) formés respectivement sur la première extrémité (110) du boîtier (101) ou sur le couvercle (120).

5. Dispositif d'atomisation (100) selon la revendication 1, dans lequel le couvercle (120) est coulissable par rapport à la première extrémité (110) du boîtier (101) afin d'exposer ou de couvrir l'orifice d'injection (111) et l'orifice d'évent (112) et comprenant en outre :
une structure de positionnement destinée à indiquer la première extrémité (110) du boîtier (101) est fermée hermétiquement par le couvercle (120) ;
dispositif dans lequel la structure de positionnement comprend deux éléments de déformation (125) et deux éléments de correspondance (114) formées respectivement sur le couvercle (120) et sur la première extrémité (110) du boîtier (101), lorsqu'une relation de correspondance est formée entre les deux éléments de déformation (125) et
les deux éléments de correspondance (114), l'endroit en gradin de la première extrémité (110) du boîtier (101) est fermé hermétiquement par le couvercle (120) ; et lorsque la relation de correspondance est relâchée, le couvercle expose au moins une partie de l'endroit en gradin de la première extrémité (110) du boîtier (101).

6. Dispositif d'atomisation (100) selon la revendication 5, dans lequel les deux éléments de déformation (125) sont formés de façon fixe sur le couvercle (120) ou sur la première extrémité (110) du boîtier (101) en étant insérés dans deux deuxièmes orifices fixes (124) formés respectivement sur le couvercle (120) ou sur la première extrémité (110) du boîtier (101).

7. Dispositif d'atomisation (100) selon la revendication 6, dans lequel les deux éléments de déformation (125) sont respectivement des douilles élastiques, et les deux éléments de correspondance (114) sont réalisés par des deux rainures de coulissement (114) formés sur la première extrémité du boîtier (101) ou sur le couvercle (120).

8. Dispositif d'atomisation (100) selon la revendication 7, dans lequel chacun des deux éléments de déformation (125) comprend un ressort (1251) et un élément supérieur (1252) disposé sur le ressort (1251).

9. Dispositif d'atomisation (100) selon la revendication 1, dans lequel une bague d'étanchéité (113) est en outre disposée sur la première extrémité (110) ou sur le couvercle (120) et autour de l'orifice d'injection (111) et de l'orifice d'évent (112) afin de former un raccordement étanche entre le couvercle (120) et la première extrémité (110) du boîtier (101) lorsque le couvercle couvre l'orifice d'injection (111) et l'orifice d'évent (112), afin d'éviter une fuite de tabac liquide.

10. Dispositif d'atomisation (100) selon la revendication 1, dans lequel l'orifice d'injection (111) présente une ouverture plus grande que celle de l'orifice d'évent (112).

11. Dispositif d'atomisation (100) selon la revendication 1, dans lequel une partie rude (121) est formée sur le couvercle (120) afin d'augmenter le toucher d'un utilisateur.

12. Cigarette électronique comprenant :
un dispositif d'atomisation (100) selon l'une quelconque des revendications précédentes 1 - 11 ; et une alimentation électrique (200), configurée pour alimenter le dispositif d'atomisation (100) en énergie électrique.
